# EUROPEAN PATENT SPECIFICATION

(11) **EP 1 651 217 B1**
(45) Date of publication and mention of the grant of the patent: **20.02.2008**
(21) Application number: 04742008.8
(22) Date of filing: 22.07.2004
(51) Int. Cl.: A61K 31/4439, A61P 1/04

(54) **ALKALINE SALTS OF PROTON PUMP INHIBITORS**
ALKALISALZE VON PROTONENPUMPEN-HEMMERN
SELS ALCALINS DES INHIBITEURS DE LA POMPE A PROTONS

(30) Priority: 23.07.2003 EP 03016759; 23.07.2003 EP 03016760
(43) Date of publication of application: 03.05.2006
(73) Proprietor: Nycomed GmbH, 78467 Konstanz (DE)
(72) Inventor: KOHL, Bernhard, 78465 Konstanz (DE); MUELLER, Bernd, 78467 Konstanz (DE); STURM, Ernst, 78465 Konstanz (DE); HUMMEL, Rolf-Peter, 78315 Radolfzell (DE)
(74) Representative: Wolf, Ulrich
(86) International application number: PCT/EP2004/051578
(87) International publication number: WO 2005/011692

(56) References cited:
- WO-A-94/25028
- DE-A- 10 061 137
- US-A- 4 738 974
- US-A- 5 693 818
- US-B1- 6 369 085
- US-B1- 6 410 569

## Description

### Subiect-matter of the invention

The present invention relates to alkaline salts of proton pump inhibitors. The novel salts can be used in the pharmaceutical industry for preparing medicaments.

### Technical background

Owing to their H⁺/K⁺-ATPase-inhibitory action, pyridin-2-ylmethylsutphinyl-1H-benzimidazoles, such as those known, for example, from EP-A-0005129, EP-A-0166287, EP-A-0174726 and EP-A-0268956, are of considerable importance in the therapy of disorders associated with an increased secretion of gastric acid.

Examples of active compounds from this group which are commercially available or in clinical development are 5-methoxy-2-[(4-methoxy-3,5-dimethyl-2-pyridinyl)methylsulphinyl]-1H-benzimidazole (INN: omeprazole), (S)-5-methoxy-2-[(4-methoxy-3,5-dimethyl-2-pyridinyl)methylsulphinyl]-1H-benzimidazole (INN: esomeprazole), 5-difluoromethoxy-2-[(3,4-dimethoxy-2-pyridinyl)methylsulphinyl]-1H-benzimidazole (INN: pantoprazole), 2-[3-methyl-4-(2,2,2-trifluoroethoxy)-2-pyridinyl)methylsulphinyl]-1H-benzimidazole (INN: lansoprazole), 2-{[4-(3-methoxypropoxy)-3-methylpyridin-2-yl]methylsulphinyl}-1H-benzimidazole (INN: rabeprazole) and 5-methoxy-2-((4-methoxy-3,5-dimethyl-2-pyridylmethyl)sulphinyl)-1H-imidazo[4,5-b]pyridine (INN: tenatoprazole).

The abovementioned sulphinyl derivatives are, owing to their mechanism of action, also referred to as proton pump inhibitors or, abbreviated, as PPI.

### Prior art

For the first time, the European Patent Application EP0080602 describes the specific preparation of a sodium and of a calcium salt of a certain pyridin-2-ylmethylsulphinyl-1H-benzimidazole compound. Later on, the European Patent Application EP0124495 (US 4738974) describes and claims novel salts of omeprazole with cations, such as the Li⁺, Na⁺, K⁺, Mg²⁺, Ca²⁺ or Ti⁴⁺ cation.

A common property of all of the abovementioned PPI is their sensitivity to acids (ultimately essential for effectiveness) which becomes apparent in their strong tendency to decompose in a neutral and in particular an acidic environment, giving rise to intensely coloured decomposition products. In the past, there has been no lack of considerable efforts, in spite of the sensitivity of the PPI to acids, to obtain stable and storable oral dosage forms comprising these PPI. A very common procedure to obtain stable oral PPI dosage forms, such as tablets, is the addition of an alkaline reacting compound, such as sodium carbonate, to the oral dosage form in order to render the micro-environment of the acid-labile PPI a pH of 7-12 (cf. European Patent 244380). Accordingly, stable and storable oral dosage forms (for example tablets or capsules) are now obtainable. However, the preparation of these oral dosage forms is relatively complicated, and with respect to the packaging too, certain complicated precautions have to be taken so that the dosage forms are sufficiently stable on storage even under extreme storage conditions (for example in tropical regions at high temperatures and high atmospheric humidity). Furthermore, in the past, there has been no lack of efforts to tailor the release of the PPI in the human body in the best possible manner to the respective requirements.

The international patent application WO92/08716 describes a chemical process, which allows pyridin-2-ylmethylsulphinyl-1H-benzimidazofes to be separated into their optical antipodes. The compounds mentioned as being prepared in an exemplary manner include, inter alia, the compounds (+)- and (-)-5-difluoromethoxy-2-[(3,4-dimethoxy-2-pyridinyl)methylsutphinyl]-1H-benzimidazole [= (+)-and (-)-pantoprazole]. The international patent application WO92/08716 mentions that the optical antipodes of the pyridin-2-ylmethylsulphinyl-1H-benzimidazoles, i.e. the (+)- and (-)-enantiomers or the (R)- and (S)-enantiomers, are useful as active compounds in medicaments for the treatment of gastrointestinal disorders. For the mode of application and the dosage of the active compounds, reference is made, inter alia, to the European patent 166 287.

During a symposium held in Montreal in September 1993, a poster of Kohl et al. was presented which showed synthesis and biological activity of enantiomers of pantoprazole.

The international patent applications WO94/24867 and WO94/25028 claim the use of the compounds (-)- and (+)-pantoprazole for treating gastric disorders in humans. Each stereoisomer is said to have medical advantages compared to the respective other stereoisomers. The descriptions also mention a number of different possible salts of the stereoisomers, and particular preference is given to the sodium salt.

In international patent application WO94/27988 (US 5693818), certain salts of (+)- and (-)-omeprazole and methods for their preparation are disclosed.

The international patent application WO97/41114 describes a certain process for preparing magnesium salts of pyridin-2-ylmethylsulphinyl-1H-benzimidazoles. What is described in an exemplary manner is, inter alia, the preparation of the magnesium salt of pantoprazole. According to the given analytical data, the salt that is prepared is pantoprazole magnesium in anhydrous form.

The international patent application WO00/10995 (US 6410569) describes the dihydrate of the magnesium salt of racemic pantoprazole.

U. S. Patent 6369085 relates to a novel form of the magnesium salt of the S-enantiomer of omeprazole trihydrate and to processes for preparing such a form of the magnesium salt of S-omeprazole and pharmaceutical compositions containing it.

The international patent application WO02/045693 (DE 10061137) describes a novel preparation, which is suitable for producing pharmaceutical dosage forms. In the new preparation the active ingredient, which is for example a PPI or a salt thereof, is present essentially uniformly dispersed in an excipient matrix composed of one or more excipients selected from the group of fatty alcohol, triglyceride, partial glyceride and fatty acid ester.

U. S. Patent 5997903 relates to oral presentation forms for pantoprazole, which consist of a core, an intermediate layer and an outer layer which is resistant to gastric juice.

The international patent application WO04/013126 describes (S)-pantoprazole magnesium and hydrates thereof.

### Description of the invention

It has now been found that alkaline reacting salts of PPI can be produced, which on account of their properties and high stability are outstandingly suited for the further processing in oral dosage forms, even without addition of another alkaline reacting compound.

Accordingly, the invention provides in a general aspect alkaline reacting salts of pyridin-2-ylmethyl-sulphinyl-1 H-benzimidazoles with H⁺/K⁺-ATPase-inhibitory activity.

According to the invention, "alkaline reacting salts" is understood to include pharmacologically compatible metal salts of pyridin-2-ylmethylsulphinyl-1 H-benzimidazoles with H⁺/K⁺-ATPase-inhibitory activity, in which at least one positive charge equivalent of the metal ion is counterbalanced by a hydroxyl ion.

According to the invention, "pyridin-2-ylmethylsulphinyl-1H-benzimidazoles with H⁺/K⁺-ATPase-inhibitory activity" is understood to include pantoprazole, omeprazole, lansoprazole, rabeprazole and tenatoprazole in racemic form, as well as the enantiomers of these compounds, such as (R)- and (S)-pantoprazole, (R)- and (S)-omeprazole, (R)- and (S)-lansoprazole, (R)- and (S)-rabeprazole and (R)-and (S)-tenatoprazole in pure form, mixtures thereof in any desired ratio, including in particular an enantiomer being substantially free of the respective other enantiomer.

In particular, the alkaline reacting salts of pyridin-2-ylmethylsulphinyl-1H-benzimidazoles with H⁺/K⁺-ATPase-inhibitory activity according to the invention can be characterized by the general formula 1

[Me]_{X}[PPI]_{Y}[OH]_{Z} (1)

in which
Me is a pharmacologically acceptable two-valued metal ion,
PPI is a compound selected from pantoprazole, omeprazole, lansoprazole, rabeprazole and tenatoprazole and their enantiomers,
OH is a hydroxyl ion,
X is a positive, whole number from 1 to 3,
Y is a positive, whole number from 1 to 5 and
Z is a positive, whole number from 1 to 5,
whereby the equation (Y + Z) = 2X applies.

More particularly, the invention provides compounds of the formula 1,
in which
Me is a pharmacologically acceptable two-valued metal ion selected from magnesium, calcium and zinc,
PPI is a compound selected from pantoprazole, omeprazole, lansoprazole and rabeprazole and their enantiomers,
OH is a hydroxyl ion,
X is the number 1 or 2,
Y is a positive, whole number from 1 to 3 and
Z is a positive, whole number from 1 to 3,
whereby the equation (Y + Z) = 2X applies.

A preferred subject of the invention are compounds of the formula 1,
in which
Me is magnesium,
PPI is a compound selected from pantoprazole, (R)-pantoprazole and (S)-pantoprazole,
OH is a hydroxyl ion,
X is the number 1 or 2,
Y is the number 1 or 3 and
Z is the number 1 or 3,
whereby the equation (Y + Z) = 2X applies.

A particular preferred subject of the invention are compounds of the formula 1,
in which
Me is magnesium,
PPI is (S)-pantoprazole, substantially free of (R)-pantoprazole,
OH is a hydroxyl ion,
X is the number 1 or 2,
Y is the number 1 or 3 and
Z is the number 1 or 3,
whereby the equation (Y + Z) = 2X applies.

It has now been found that the sodium salt of (-)- or (S)-pantoprazole, which is particularly preferred in the international patent application WO 94/24867, does not form a stable storage form. During attempts to obtain a stable oral dosage form for (-)-pantoprazole, it has now been found that alkaline reacting magnesium salts of (S)-pantoprazole, in particular in hydrate form, have highly surprising stability properties, making them particularly suitable candidates for use in solid or oral dosage forms. Compared to the sodium salt of (-)-pantoprazole, they have considerably improved stability properties. Thus, for example, the compound of formula 1, in which Me is magnesium, PPI is (S)-pantoprazole, X is 1, Y is 1 and Z is 1 in its hydrate form is, at 70°C, completely stable for one week and shows virtually no discolouration or decomposition, whereas over the same period of time and under identical conditions, the colour of the hydrate of (-)-pantoprazole sodium changes to brown, with formation of considerable amounts of decomposition products. In addition, the compound of formula 1, in which Me is magnesium, PPI is (S)-pantoprazole, X is 1, Y is 1 and Z is 1 in its hydrate form shows a surprising and unexpected faster dissolution than the racemic pantoprazole magnesium dihydrate.

The compounds according to the invention and their hydrates can be used for the treatment and prevention of all disorders, which can be treated or prevented by using PPI. In particular, the compounds according to the invention and their hydrates can be used for treating gastric disorders. In this context, particular mention should be made of the relatively high stability of compounds according to the invention and their hydrates. For example, on storage under atmospheric conditions, the sum of by-products in [Mg][(S)-pantoprazole][OH]xH₂O remains virtually constant, whereas in the case of (-)-pantoprazole sodium, under identical conditions (storage at 60-70°C) the purity (according to HPLC) decreases from 99.5 to 96-97 %. This relatively high storage stability makes the compounds according to the invention and their hydrates particularly suitable for use in medicaments.

The compounds according to the invention and their hydrates are prepared in a specific manner by reacting the PPI with a Me-salt in the presence of an alkali-hydroxide, or from a readily soluble PPI salt (for example (-)-pantoprazole sodium), using for example a magnesium salt, such as magnesium chloride, a sodium hydroxide solution, in water or in mixtures of water with polar organic solvents (for example alcohols, preferably methanol, ethanol or isopropanol, or ketones, preferably acetone).

Me-salts suitable for use in the process are, for example, Me-chloride, Me-bromide, Me-fluoride, Me-iodide, Me-formate, Me-acetate, Me-propionate, Me-gluconate or Me-carbonate. It is also possible to react Me-alkoxides (for example Me-methoxide, Me-ethoxide, Me-(iso)propoxide, Me-butoxide, Me-hexoxide or Me-phenoxide) in alkoholate medium with a readily soluble PPI salt in the presence of an alkali-hydroxide solution and to crystallise the alkaline PPI salt in its hydrate form by addition of water. Furthermore it is possible to recrystallise the compounds according to the invention and their hydrates from e.g. methanol/water mixtures.

The examples below illustrate the invention in more detail. m. p. denotes melting point, min. denotes minute(s), h denotes hour(s).

### Examples

### 1. Magnesium {[5-(difluoromethoxy)]-2-[(3,4-dimethoxy-2-pyridinyl)methylsulphinyl]-1H-benzimidazolidel} hydroxy monohydrate

### Formula [Pantoprazole⁻OH⁻Mg²⁺H₂O]: C₁₆H₁₇F₂N₃O₆SMg

10.7 g of pantoprazole sodium sesquihydrate are dissolved in 110 ml of water. 2.48 g of NaOH 40 % are added at 25 °C and the solution is stirred for 1 h. 5.01 g of magnesium chloride hexahydrate are dissolved in 20 ml of water, the solution is added drop wise under stirring at 25 °C to the pantoprazole sodium salt solution. Stirring is continued for 1 h. The resulting suspension is filtered with suction, the precipitate washed with 50 ml of water. The precipitate is re-suspended in 100 ml of water and again filtered and dried in a vacuum dryer (< 50 mbar) at 40-45°C to give 10.76 g (94.6 %) of the title compound of m. p. 184-187 °C (decomposition) as off-white solid.
Analysis:

| | expected | found |
|---|---|---|
| C | 43,51 | 43,87 |
| H | 3,88 | 4,10 |
| N | 9,51 | 9,68 |
| Mg | 5,50 | 5,2 |

### 2. Magnesium (-)-{[5-(difluoromethoxy)]-2-[(3,4-dimethoxy-2-pyridinyl)methylsulphinyl]-1H-benzimidazolide} hydroxy monohydrate

### Formula [(S)-Pantoprazole⁻OH⁻Mg²⁺H₂O]): C₁₆H₁₇F₂N₃O₆SMg

10.9 g of (-)-pantoprazole sodium salt are dissolved in 110 ml of water. 2.48 g of NaOH 40 % are added at 50 °C and the solution is stirred for 1 hour. The solution is cooled to room temperature. 5.01 g of magnesium chloride hexahydrate are dissolved in 20 ml of water. The magnesium chloride solution is added drop wise under stirring at 25 °C to the (-)-pantoprazole sodium salt solution. Stirring is continued for 18 h. The resulting suspension is filtered with suction, the precipitate washed with 50 ml of water. The precipitate is re-suspended in 100 ml of water and again filtered and dried in a vacuum dryer (< 50 mbar) at 40 - 45 °C to give 10.01 g (88.0 %) of the title compound of m. p. 164-167 °C (decomposition) as off-white solid
Specific rotation: α_{D}^{20°} = -123 (c= 0.5, methanol)
Analysis:

| | expected | found |
|---|---|---|
| C | 43.51 | 43.66 |
| H | 3.88 | 4.16 |
| N | 9.51 | 9.63 |
| Mg | 5.50 | 5.6 |

### 3. Magnesium (+)-{[5-(difluoromethoxy)]-2-[(3,4-dimethoxy-2-pyridinyl)methylsulphinyl]-1H-benzimidazolide} hydroxy sesquihydrate

### Formula [2 (R)-Pantoprazole⁻2OH⁻2Mg²⁺3H₂O]): C₃₂H₃₃F₄N₆O₁₁S₂Mg₂

5.0 g of (+)-pantoprazole sodium salt are dissolved in 50 ml of water. 1.23 g of NaOH 40 % are added at 35 °C and the solution is stirred for 1 h. The solution is cooled to room temperature. 2.50 g of magnesium chloride hexahydrate are dissolved in 15 ml of water. The magnesium chloride solution is added drop wise under stirring at 25 °C to the (+)-pantoprazole sodium salt solution. Stirring is continued for 18 h. The resulting suspension is filtered with suction, the precipitate washed in 3 portions with 50 ml of water and dried in an vacuum dryer (< 50 mbar) at 50 - 55 °C to give 4.33 g (74.6 %) of the title compound of m. p. 161-165 °C (decomposition) as off-white solid
Specific rotation: α_{D}^{20°} = +112 (c= 0.5, methanol)
Analysis:

| | expected | found |
|---|---|---|
| C | 42.64 | 42.44 |
| H | 4.03 | 4.02 |
| N | 9.32 | 9.31 |

### 4. Di-Magnesium tris-{[5-(difluoromethoxy)]-2-[(3,4-dimethoxy-2-pyridinyl)methylsulphinyl]-1H-benzimidazolide}hydroxy tetrahydrate

### Formula [3 Pantoprazole⁻OH⁻2Mg²⁺4H₂O]: C₄₈H₅₁F₆N₉O₁₇S₃Mg₂

25.0 g of pantoprazole sodium sesquihydrate are dissolved in 250 ml of water. 1.33 ml of NaOH 40 % are added at 25 °C and the solution is stirred for 15 min. 3.92 g of magnesium chloride hexahydrate are dissolved in 31 ml of water. The magnesium chloride solution is added drop wise under stirring at 25 °C to the pantoprazole sodium salt solution. Stirring is continued for 2.5 h. The resulting suspension is filtered with suction, the precipitate washed in 3 portions with 150 ml of water. The precipitate is dried in a vacuum dryer (< 50 mbar) at 40 - 45 °C to give 12.47 g (52.7 %) of the title compound of m. p. 182-185 °C (decomposition) as off-white solid.
Analysis:

| | expected* | found |
|---|---|---|
| C | 44,87 | 45,50 |
| H | 4,00 | 4,03 |
| N | 9,81 | 10,01 |
| Mg | 3,78 | 3,9 |

| | | |
|---|---|---|
| *as tetrahydrate | | |

### 5. Di-Macinesium (-)-tris-{[5-(difluoromethoxy)]-2-[(3,4-dimethoxy-2-pyridinyl)methylsulphinyl]-1H-benzimidazolide} hydroxy pentahydrate

### Formula [3 (S)-Pantoprazole⁻OH⁻2Mg²⁺5H₂O]: C₄₈H₅₃F₆N₉O₁₈S₃Mg₂

6.0 g of (-)-pantoprazole sodium are dissolved in 60 ml of water. 0.49 g of NaOH 40 % are added at 30 - 35 °C and stirred for 15 min. 1.02 g of magnesium chloride hexahydrate are dissolved in 8 ml of water. The magnesium chloride solution is added drop wise under stirring at 25 °C to the (-)-pantoprazole sodium salt solution. Stirring is continued for 18 h. The resulting suspension is filtered with suction, the precipitate washed in 2 portions with 50 ml of water. The precipitate is dried in a vacuum dryer (< 50 mbar) at 60 °C to give 2.41 g (48.3 %) of the title compound of m. p. 162-166°C (decomposition) as off-white solid.
Specific rotation: α_{D}^{20°} = -125 (c= 0,5 in Methanol)
Analysis:

| | expected* | found |
|---|---|---|
| C | 44,25 | 44,34 |
| H | 4,10 | 4,29 |
| N | 9,68 | 9,80 |

| | | |
|---|---|---|
| *as pentahydrate | | |

### 6. Di-Magnesium (+)-tris-{[5-(difluoromethoxy)]-2-[(3,4-dimethoxy-2-pvridinyl)methylsulphinyl]-1H-benzimidazolide} hydroxy pentahydrate

### Formula [3 (R)-Pantoprazole⁻OH⁻2Mg²⁺5H₂O]: C₄₈H₅₃F₆N₉O₁₈S₃Mg₂

5.0 g of (+)-pantoprazole sodium are dissolved in 50 ml of water. 0.27 ml of NaOH 40 % are added at 30 - 35 °C and the solution is stirred for 15 min. 0.78 g of magnesium chloride hexahydrate are dissolved in 6 ml of water. The magnesium chloride solution is added drop wise under stirring at 25 °C to the (+)-pantoprazole sodium salt solution. Stirring is continued for 2 days. The resulting suspension is filtered with suction, the precipitate washed in 3 portions with 25 ml of water. The precipitate is dried in a vacuum dryer (< 50 mbar) at 40 °C to give 2.10 g (40.1 %) of the title compound of m. p. 161-166°C (decomposition)as off-white solid.
Specific rotation: α_{D}^{20°} = +114,5 (c= 0.5 in Methanol)
Analysis:

| | expected* | found |
|---|---|---|
| C | 44,25 | 44,81 |
| H | 4,10 | 4,05 |
| N | 9,68 | 9,79 |
| Mg | 3,73 | 4,2 |

| | | |
|---|---|---|
| *as pentahydrate | | |

### 7. Calcium {[5-(difluoromethoxy)]-2-[(3,4-dimethoxy-2-pyridinyl)methylsulphinyl]-1H-benzimidazolide}hydroxy monohydrate

### Formula [Pantoprazole⁻OH⁻Ca²⁺H₂O]: C₁₆H₁₇F₂N₃O₆SCa

21.6 g of pantoprazole sodium sesquihydrate are dissolved in 250 ml of water. 2.00 g of NaOH are added at 25 °C and the solution is stirred for 1 hour. 5.55g of calcium chloride (dry) are dissolved in 50 ml of water. The calcium chloride solution is added drop by drop under stirring at 25 °C to the pantoprazole sodium solution. Stirring is continued for 20 hours. The resulting suspension is filtered with suction, the precipitate is washed with 200 ml of water and dried in a vacuum dryer (< 50 mbar) at 40 - 45 °C to afford 21.86 g (91.8 %) of the title compound as off-white solid.

| | |
|---|---|
| Water (Karl-Fischer titration): | 7.6 % |
| Melting point: | 157-160 °C (decomposition) |

Analysis:

| | expected | found |
|---|---|---|
| C | 42.01 | 42,63 |
| H | 3.75 | 3.95 |
| N | 9.19 | 9.13 |

### 8. Calcium (-)-{[5-(difluoromethoxy)]-2-[(3,4-dimethoxy-2-pyridinyl)methylsulphinyl]-1H-benzimidazolide} hydroxy monohydrate

### Formula [Pantoprazole⁻OH⁻Ca²⁺H₂O]: C₁₆H₁₇F₂N₃O₆SCa

22.7 g of (-)-pantoprazole sodium (wet, 0.05 mol) are dissolved in 250 ml of water. 2.00 g of NaOH are added at 25°C and the solution is stirred for 1 hour at 40 °C. 5.55g of calcium chloride (dry) are dissolved in 50 ml of water. The calcium chloride solution is added drop by drop under stirring at 25 °C to the pantoprazole sodium solution. Stirring is continued for 20 hours. The resulting suspension is filtered with suction, the precipitate is washed with 200 ml of water and dried in a vacuum dryer (< 50 mbar) at 40 - 45 °C to afford 21.44 g (89.9 %) of the title compound as off-white solid.

| | |
|---|---|
| Water (Karl-Fischer titration): | 7.8 % |
| Melting point: | 137-147 °C (decomposition) |

Analysis:

| | expected | found |
|---|---|---|
| C | 42.01 | 41.74 |
| H | 3.75 | 3.81 |
| N | 9.19 | 8.99 |

### 9. Zinc {[5-(difluoromethoxy)]-2-[(3,4-dimethoxy-2-pyridinyl)methylsulphinyl]-1H-benzimidazolide} hydroxy monohydrate

### Formula [Pantoprazole⁻OH⁻Zn²⁺H₂O]: C₁₆H₁₇F₂N₃O₆SZₙ

21.6 g of pantoprazole sodium sesquihydrate are dissolved in 250 ml of water. 2.00 g of NaOH are added at 25 °C and the solution is stirred for 1 hour. 6.80 g of zinc chloride (dry) are dissolved in 50 ml of water. The zinc chloride solution is added drop by drop under stirring at 25 °C to the pantoprazole sodium solution. Stirring is continued for 20 hours. The resulting suspension is filtered with suction, the precipitate is washed with 200 ml of water and dried in a vacuum dryer (< 50 mbar) at 40 - 45 °C to afford 23.08 g (94.2 %) of the title compound as off-white solid.

| | |
|---|---|
| Water (Karl-Fischer titration): | 5.1 % |
| Melting point: | 167-179 °C (decomposition) |

Analysis:

| | expected | found |
|---|---|---|
| C | 39.81 | 40.55 |
| H | 3.55 | 3.25 |
| N | 8.70 | 8.74 |

### 10. Zinc (-)-{[5-(difluoromethoxy)]-2-[(3,4-dimethoxy-2-pyridinyl)methylsulphinyl]-1H-benzimidazolide}hydroxy monohydrate

### Formula [(S)-Pantoprazole⁻OH⁻Zn²⁺H₂O]: C₁₆H₁₇F₂N₃O₆SZn

22.7 g of (-)-pantoprazole sodium (wet, 0.05 mol) are dissolved in 250 ml of water. 2.00 g of NaOH are added at 25 °C and the solution is stirred for 1 hour. 6.80 g of zinc chloride (dry) are dissolved in 50 ml of water. The zinc chloride solution is added drop by drop under stirring at 25 °C to the pantoprazole sodium solution. Stirring is continued for 20 hours. The resulting suspension is filtered with suction, the precipitate is washed with 200 ml of water and dried in a vacuum dryer (< 50 mbar) at 40-45 °C to afford 22.87 g (94.2 %) of the title compound as off-white solid.

| | |
|---|---|
| Water (Karl-Fischer titration): | 4.3 % |
| Melting point: | 169-173 °C (decomposition) |

Analysis:

| | expected | found |
|---|---|---|
| C | 39.81 | 40.81 |
| H | 3.55 | 3.24 |
| N | 8.70 | 8.80 |

### 11. Magnesium (-)-{[5-(difluoromethoxy)]-2-[(3,4-dimethoxy-2-pyridinyl)methylsulphinyl]-1H-benzimidazolide} hydroxy monohydrate

### Formula [(S)-Pantoprazole⁻OH⁻Mg²⁺H₂O]: C₁₆H₁₇F₂N₃O₆SMg

6.20 kg of (-)-pantoprazole sodium (wet, 14.06 mol) are dissolved in 64 I of water. 0.56 kg of NaOH are added at room temperature and the solution is stirred for 1 hour at 40 °C. The solution is cooled to room temperature. 2.86 kg of magnesium chloride hexahydrate are dissolved in 11.4 I of water. The magnesium chloride solution is added under stirring at 25 °C to the (-)-pantoprazole sodium solution. Stirring is continued for 18 hours. The resulting suspension is centrifuged, the precipitate is washed with 41 I of water and dried in a vacuum dryer (< 50 mbar) at 40 -45 °C to afford 3.97 kg (58.84 % of the title compound as off-white solid).

| | |
|---|---|
| Purity (HPLC): | 99.4 % |
| ee: | >99 % |
| Water (Karl-Fischer titration): | 7.7 % |

Analysis:

| | expected | found |
|---|---|---|
| C | 43.51 | 43.05 |
| H | 3.88 | 3.97 |
| N | 9.51 | 9.35 |
| S | 7.26 | 7.24 |

### Commercial utility

The compounds according to the invention and their hydrates have useful pharmacological properties, rendering them commercially utilizable. In particular, they have a pronounced inhibitory effect on the secretion of gastric acid and excellent gastrointestinal protective action in warm-blooded animals, in particular man. Due to their unique stability characteristics, the compounds according to the invention and their hydrates are particularly suited for the production of stable oral PPI dosage forms. By the presence of the hydroxyl ion in the compounds according to the invention, the PPI, which are acid-labile and susceptible to traces of acid, have a self-protection against undesired decomposition on storage.

In context of the invention, "gastrointestinal protection" is to be understood as the prevention and treatment of gastrointestinal disorders, in particular gastrointestinal inflammatory disorders and lesions (such as, for example, Ulcus ventriculi, Ulcus duodeni, gastritis, irritable bowel owing to an increased production of acid or as a result of medicaments, GERD, Crohn's disease, IBD) which may be caused, for example, by microorganisms (for example Helicobacter pylori), bacterial toxins, medicaments (for example certain antiphlogistics and antirheumatic drugs), chemicals (for example ethanol), gastric acid or stress.

With their excellent properties, selected compounds according to the invention and their hydrates are, in various models for the determination of antiulcerogenic and antisecretory properties, surprisingly clearly superior to the prior-art compounds, in particular with respect to their stability and their pharmacological properties. Owing to these properties, the compounds [Mg][(S)-pantoprazole][OH]xH₂O and [Mg]₂[(S)-pantoprazole]₃[OH]xH₂O for example are highly suitable for use in human and veterinary medicine, where they are used, in particular, for the treatment and/or prophylaxis of gastrointestinal disorders.

Accordingly, the invention furthermore provides compounds according to the invention and their hydrates for use in the treatment and/or prophylaxis of the abovementioned diseases.

The invention also embraces the use of compounds according to the invention and their hydrates for preparing medicaments used for the treatment and/or prophylaxis of the abovementioned diseases.

The invention also provides medicaments comprising compounds according to the invention and their hydrates.

The medicaments are prepared by processes known per se which are familiar to the person skilled in the art. As medicaments, the compounds according to the invention and their hydrates are employed either as such or, preferably, in combination with suitable pharmaceutical auxiliaries or carriers in the form of tablets, coated tablets, capsules, suppositories, plasters (for example as TTS), emulsions, suspensions or solutions, where the content of active compound is advantageously from 0.1 to 95% and where it is possible to produce pharmaceutical dosage forms (for example flow-release forms or enteric forms) which, by the appropriate choice of auxiliaries and carriers, are tailored for the active compound and/or the desired onset of action and/or the duration of action.

The auxiliaries or carriers suitable for the desired pharmaceutical formulations are known to the person skilled in the art owing to his expert knowledge. In addition to solvents, gel formers, suppository bases, tabletting auxiliaries and other carriers for active compounds, it is possible to use, for example, antioxidants, dispersants, emulsifiers, antifoams, flavour-masking agents, preservatives, solubilizers, colorants or, in particular, permeation promoters and complex formers (for example cyclodextrins).

The compounds according to the invention and their hydrates can be administered orally, parenterally or percutaneously.

In human medicine, it has generally been found to be advantageous to administer the compounds according to the invention and their hydrates, when given orally, in a daily dose of from about 0.1 to 2, preferably 0.2 to 1.5 and in particular 0.3 to 1.1, mg/kg of body weight [based on the PPi], if appropriate in the form of a plurality of, preferably 1 to 4, individual doses, to obtain the desired result. For parenteral treatment, it is possible to use similar or (in particular when the active compounds are administered intravenously) generally lower dosages. The optimum dosage and the type of administration of the active compounds required in each case can easily be determined by the person skilled in the art, owing to his expert knowledge.

A further aspect of the invention is thus a medicament, comprising a compound according to the invention or its hydrate together with customary auxiliaries, where the single dose comprises from 10 to 100 mg of PPI.

A further aspect of the invention is a medicament, comprising a compound according to the invention or its hydrate together with customary auxiliaries, where the single dose comprises from 20 to 80 mg of (-)-pantoprazole.

A further aspect of the invention is the use of a compound according to the invention or its hydrate for treating gastrointestinal disorders.

A further aspect of the invention is the use of a compound according to the invention or its hydrate for treating gastrointestinal disorders in patients who are slow metabolizers.

A further aspect of the invention is the use of a compound according to the invention or its hydrate for treating gastrointestinal disorders in patients who have a risk for drug interactions.

A further aspect of the invention is the use of a compound according to the invention or its hydrate for treating gastrointestinal disorders in patients who need an inhibition of acid secretion for a longer period of time.

A further aspect of the invention is a medicament for treating gastrointestinal disorders for use in patients who are slow metabolizers, comprising a compound according to the invention or its hydrate together with customary auxiliaries, where the single dose comprises from 10 to 100 mg of the PPI.

A further aspect of the invention is a medicament for treating gastrointestinal disorders for use in patients who are slow metabolizers, comprising a compound according to the invention or its hydrate together with customary auxiliaries, where the single dose comprises from 20 to 80 mg of the PPI.

A further aspect of the invention is a medicament for treating gastrointestinal disorders for use in patients who have a risk for drug interactions, comprising a compound according to the invention or its hydrate together with customary auxiliaries, where the single dose comprises from 10 to 100 mg of the PPI.

A further aspect of the invention is a medicament for treating gastrointestinal disorders for use in patients who have a risk for drug interactions, comprising a compound according to the invention or its hydrate together with customary auxiliaries, where the single dose comprises from 20 to 80 mg of the PPI.

A further aspect of the invention is a medicament for treating gastrointestinal disorders for use in patients who need an inhibition of acid secretion for a longer period of time, comprising a compound according to the invention or its hydrate together with customary auxiliaries, where the single dose comprises from 10 to 100 mg of the PPI.

A further aspect of the invention is a medicament for treating gastrointestinal disorders for use in patients who need an inhibition of acid secretion for a longer period of time, comprising a compound according to the invention or its hydrate together with customary auxiliaries, where the single dose comprises from 20 to 80 mg of the PPI.

If a compound according to the invention or its hydrate is to be used for treating the abovementioned diseases, the pharmaceutical preparations may also comprise one or more pharmacologically active ingredients from other groups of medicaments. Examples which may be mentioned are: tranquilizers (for example from the group of the benzodiazepines, e. g. diazepam), spasmolytic drugs (e. g. bietamiverine or camylofine), anticholinergic drugs (e. g. oxyphencyclimine or phencarbamide), local anesthetics (e. g. tetracaine or procaine), optionally also enzymes, vitamins or amino acids.

In this context, particular emphasis is given to the combination of the compounds according to the invention with other pharmaceuticals which buffer or neutralize gastric acid or which inhibit the secretion of acid, such as, for example, antacids (such as, for example, magaldrate) or H₂ blockers (e. g. cimetidine, ranitidine), and with gastrin antagonists with the aim to enhance the main action in an additive or superadditive sense and/or to eliminate or reduce side-effects or to obtain a more rapid onset of action. Mention may also be made of the fixed or free combination with NSAIDs (such as, for example, etofenamate, diclofenac, indometacin, ibuprofen or piroxicam) for preventing the gastrointestinal damage caused by the NSAIDs, or with antibacterial substances (such as, for example, cephalosporins, tetracyclins, penicillins, macrolides, nitroimidazoles or else bismuth salt) for controlling Helicobacter pylori. Antibacterial combination partners which may be mentioned are, for example, meziocillin, ampicillin, amoxicillin, cefalothin, cefoxitin, cefotaxim, imipenem, gentamycin, amicacin, erythromycin, ciprofloxacin, metronidazole, clarithromycin, azithromycin and combinations thereof (e. g. clarithromycin + metronidazole or amoxicillin + clarithromycin).

## Claims

1. Pharmacologically compatible metal salts of pyridin-2-ylmethylsulphinyl-1H-benzimfdazoles with H⁺/K⁺-ATPase-inhibitory activity, in which at least one positive charge equivalent of the metal ion is counterbalanced by a hydroxyl ion, and hydrates thereof.

2. Pharmacologically compatible metal salts according to claim 1, in which the pyridin-2-ylmethyl-sulphinyl-1 H-benzimidazoles with H⁺/K⁺-ATPase-inhibitory activity is selected from the group of pantoprazole, omeprazole, lansoprazole, rabeprazole and tenatoprazole, (R)- and (S)-pantoprazole. (R)- and (S)-omeprazole, (R)- and (S)-lansoprazole, (R), and (S)-rabeprazole and (R)- and (S)-tenatoprazole, and hydrates thereof.

3. Pharmacologically compatible metal salts according to claim 1, **characterized by** the general formula 1
[Me]_{X}[PPI]_{Y}[OH]_{Z} (1)
in which
Me is a pharmacologically acceptable two-valued metal ion,
PPI is a compound selected from pantoprazole, omeprazole, lansoprazole, rabeprazole and tenatoprazole and their enantiomers,
OH is a hydroxyl ion,
X is a positive, whole number from 1 to 3,
Y is a positive, whole number from 1 to 5 and
Z is a positive, whole number from 1 to 5,
whereby the equation (Y + Z) = 2X applies, and hydrates thereof.

4. Pharmacologically compatible metal salts according to claim 1, **characterized by** the general formula 1 of claim 3, in which
Me is a pharmacologically acceptable two-valued metal ion selected from magnesium, calcium and zinc,
PPI is a compound selected from pantoprazole, omeprazole, lansoprazole, rabeprazole and tenatoprazole and their enantiomers,
OH is a hydroxyl ion,
X is the number 1 or 2,
Y is a positive, whole number from 1 to 3 and
Z is a positive, whole number from 1 to 3.
whereby the equation (Y + Z) = 2X applies, and hydrates thereof.

5. Pharmacologically compatible metal salts according to claim 1, **characterized by** the general formula 1 of claim 3, in which
Me is magnesium,
PPI is a compound selected from pantoprazole, (R)-pantoprazole and (S)-pantoprazole,
OH is a hydroxyl ion,
X is the number 1 or 2,
Y is the number 1 or 3 and
Z is the number 1 or 3,
whereby the equation (Y + Z) = 2X applies, and hydrates thereof.

6. Pharmacologically compatible metal salts according to claim 1, **characterized by** the general formula 1 of claim 3, in which
Me is magnesium,
PPI is (S)-pantoprazole,
OH is a hydroxyl ion,
X is the number or 2,
Y is the number 1 or 3 and
Z is the number 1 or 3,
whereby the equation (Y + Z) = 2X applies, and hydrates thereof.

7. Pharmacologically compatible metal salt according to claim 1, which is Mg[Pantoprazole]OH, and hydrates thereof.

8. Pharmacologically compatible metal salt according to claim 1, which is Mg[(S)-Pantoprazole]OH, and hydrates thereof.

9. Pharmacologically compatible metal salt according to claim 1, which is Mg₂[Pantoprazole]₃OH, and hydrates thereof.

10. Pharmacologically compatible metal salt according to claim 1, which is Mg₂[(S)-Pantoprazole]₃OH, and hydrates thereof.

11. Medicament, comprising a compound according to any of claims 1 to 10 together with customary auxiliaries.

12. Use of a compound as claimed in any of claims 1 to 10 for the preparation of a medicament for treating gastrointestinal disorders.

## Patentansprüche

1. Pharmakologisch verträgliche Metallsalze von Pyridin-2-ylmethylsulfinyl-1H-benzimidazolen mit H⁺/K⁺-ATPase-hemmender Wirkung, bei denen wenigstens ein positives Ladungsäquivalent des Metallions durch ein Hydroxylion kompensiert wird, und Hydrate davon.

2. Pharmakologisch verträgliche Metallsalze nach Anspruch 1, bei denen die Pyridin-2-ylmethylsulfinyl-1H-benzimidazole mit H⁺/K⁺-ATPase-hemmender Wirkung ausgewählt sind aus der Gruppe bestehend aus Pantoprazol, Omeprazol, Lansoprazol, Rabeprazol und Tenatoprazol, (R)- und (S)-Pantoprazol, (R)- und (S)-Omeprazol, (R)- und (S)-Lansoprazol, (R)- und (S)-Rabeprazol und (R)- und (S)-Tenatoprazol, und Hydraten davon.

3. Pharmakologisch verträgliche Metallsalze nach Anspruch 1, **gekennzeichnet durch** die allgemeine Formel I
[Me]ₓ[PPI]_{y}[OH]_{z} (1)
in welcher
Me für ein pharmakologisch unbedenkliches zweiwertiges Metallion steht,
PPI für eine Verbindung ausgewählt aus Pantoprazol, Omeprazol, Lansoprazol, Rabeprazol und Tenatoprazol und ihren Enantiomeren steht,
OH für ein Hydroxylion steht,
X für eine positive ganze Zahl von 1 bis 3 steht,
Y für eine positive ganze Zahl von 1 bis 5 steht und
Z für eine positive ganze Zahl von 1 bis 5 steht,
wobei die Gleichung (Y + Z) = 2X zutrifft, und Hydrate davon.

4. Pharmakologisch verträgliche Metallsalze nach Anspruch 1, **gekennzeichnet durch** die allgemeine Formel I nach Anspruch 3, in welcher
Me für ein pharmakologisch unbedenkliches zweiwertiges Metallion ausgewählt aus Magnesium, Calcium und Zink steht,
PPI für eine Verbindung ausgewählt aus Pantoprazol, Omeprazol, Lansoprazol, Rabeprazol und Tenatoprazol und ihren Enantiomeren steht,
OH für ein Hydroxylion steht,
X für die Zahl 1 oder 2 steht,
Y für eine positive ganze Zahl von 1 bis 3 steht und
Z für eine positive ganze Zahl von 1 bis 3 steht,
wobei die Gleichung (Y + Z) = 2X zutrifft, und Hydrate davon.

5. Pharmakologisch verträgliche Metallsalze nach Anspruch 1, **gekennzeichnet durch** die allgemeine Formel I nach Anspruch 3, in welcher
Me für Magnesium steht,
PPI für eine Verbindung ausgewählt aus Pantoprazol, (R)-Pantoprazol und (S)-Pantoprazol steht,
OH für ein Hydroxylion steht
X für die Zahl 1 oder 2 steht,
Y für die Zahl 1 oder 3 steht und
Z für die Zahl 1 oder 3 steht,
wobei die Gleichung (Y + Z) = 2X zutrifft, und Hydrate davon.

6. Pharmakologisch verträgliche Metallsalze nach Anspruch 1, **gekennzeichnet durch** die allgemeine Formel I nach Anspruch 3, in welcher
Me für Magnesium steht,
PPI (S)-Pantoprazol steht,
OH für ein Hydroxylion steht
X für die Zahl 1 oder 2 steht,
Y für die Zahl 1 oder 3 steht und
Z für die Zahl 1 oder 3 steht,
wobei die Gleichung (Y + Z) = 2X zutrifft, und Hydrate davon.

7. Pharmakologisch verträgliches Metallsalz nach Anspruch 1, bei dem es sich um Mg[Pantoprazol]OH handelt, und dessen Hydrate.

8. Pharmakologisch verträgliches Metallsalz nach Anspruch 1, bei dem es sich um Mg[(S)-Pantoprazol]OH handelt, und dessen Hydrate.

9. Pharmakologisch verträgliches Metallsalz nach Anspruch 1, bei dem es sich um Mg₂[Pantoprazol]₃OH handelt, und dessen Hydrate.

10. Pharmakologisch verträgliches Metallsalz nach Anspruch 1, bei dem es sich um Mg₂[(S)-Pantoprazol]₃OH handelt, und dessen Hydrate.

11. Arzneimittel, enthaltend eine Verbindung nach einem der Ansprüche 1 bis 10 zusammen mit herkömmlichen Hilfsstoffen.

12. Verwendung einer Verbindung nach einem der Ansprüche 1 bis 10 zur Herstellung eines Medikaments zur Behandlung von Erkrankungen des Magen-Darm-Trakts.

## Revendications

1. Sels de métal pharmacologiquement compatibles de pyridin-2-ylméthylsulfinyl-1H-benzimidazoles ayant une activité inhibitrice de H⁺/K⁺ ATPase, dans lesquels au moins une charge positive équivalente de l'ion de métal est contrebalancée par un ion hydroxyle, et des hydrates de ceux-ci.

2. Sels de métal pharmacologiquement compatibles selon la revendication 1, dans lesquels le pyridin-2-ylméthylsulfinyl-1H-benzimidazole ayant une activité inhibitrice de H⁺/K⁺ ATPase est choisi dans le groupe constitué du pantoprazole, de l'oméprazole, du lansoprazole, du rabéprazole et du ténatoprazole, du (R)- et (S)-pantoprazole, du (R)- et (S)-oméprazole, du (R)- et (S)-lansoprazole, du (R)- et (S)-rabéprazole et du (R)- et (S)-ténatoprazole, et des hydrates de ceux-ci.

3. Sels de métal pharmacologiquement compatibles selon la revendication 1, **caractérisés par** la formule générale 1
[Me]_{X}[PPI]_{Y}[OH]_{Z} (1)
dans laquelle
Me est un ion métallique à deux valeurs pharmacologiquement acceptable,
PPI est un composé choisi parmi le pantoprazole, l'oméprazole, le lansoprazole, le rabéprazole et le ténatoprazole et leurs énantiomères,
OH est un ion hydroxyle,
X est un nombre entier positif de 1 à 3,
Y est un nombre entier positif de 1 à 5 et
Z est un nombre entier positif de 1 à 5,
où l'équation (Y + Z) = 2X s'applique, et des hydrates de ceux-ci.

4. Sels de métal pharmacologiquement compatibles selon la revendication 1, **caractérisés par** la formule générale 1 de la revendication 3, dans lesquels
Me est un ion métallique à deux valeurs pharmacologiquement acceptable choisi parmi le magnésium, le calcium et le zinc,
PPI est un composé choisi parmi le pantoprazole, l'oméprazole, le lansoprazole, le rabéprazole et le ténatoprazole et leurs énantiomères,
OH est un ion hydroxyle,
X est le nombre 1 ou 2,
Y est un nombre entier positif de 1 à 3, et
Z est un nombre entier positif de 1 à 3,
où l'équation (Y + Z) = 2X s'applique, et des hydrates de ceux-ci.

5. Sels de métal pharmacologiquement compatibles selon la revendication 1, **caractérisés par** la formule générale 1 de la revendication 3, dans lesquels
Me est le magnésium,
PPI est un composé choisi parmi le pantoprazole, le (R)-pantoprazole et le (S)-pantoprazole,
OH est un ion hydroxyle,
X est le nombre 1 ou 2,
Y est le nombre 1 ou 3 et
Z est le nombre 1 ou 3,
où l'équation (Y + Z) = 2X s'applique, et des hydrates de ceux-ci.

6. Sels de métal pharmacologiquement compatibles selon la revendication 1, **caractérisés par** la formule générale 1 de la revendication 3, dans lesquels
Me est le magnésium,
PPI est le (S)-pantoprazole,
OH est un ion hydroxyle,
X est le nombre 1 ou 2,
Y est le nombre 1 ou 3 et
Z est le nombre 1 ou 3,
où l'équation (Y + Z) = 2X s'applique, et des hydrates de ceux-ci.

7. Sel de métal pharmacologiquement compatible selon la revendication 1, qui est Mg[pantoprazole]OH, et des hydrates de celui-ci.

8. Sel de métal pharmacologiquement compatible selon la revendication 1, qui est Mg[(S)-pantoprazole]OH, et des hydrates de celui-ci.

9. Sel de métal pharmacologiquement compatible selon la revendication 1, qui est Mg₂[pantoprazole]₃OH, et des hydrates de celui-ci.

10. Sel de métal pharmacologiquement compatible selon la revendication 1, qui est Mg₂[(S)-pantoprazole]₃OH, et des hydrates de celui-ci.

11. Médicament comprenant un composé selon l'une quelconque des revendications 1 à 10 conjointement avec des excipients usuels.

12. Utilisation d'un composé selon l'une quelconque des revendications 1 à 10 pour la préparation d'un médicament pour traiter des troubles gastro-intestinaux.
